# EUROPEAN PATENT APPLICATION

(11) **EP 2 218 471 A1**
(43) Date of publication of application: **18.08.2010**
(21) Application number: 08854903.5
(22) Date of filing: 18.11.2008
(51) Int. Cl.: A61M 1/14

(54) **BLOOD DIALYSIS APPARATUS**

(30) Priority: 27.11.2007 JP 2007305528
(71) Applicant: JMS Co., Naka-ku Hiroshima-shi Hiroshima 730-8652 (JP); Kitakyushu Institute Of Biophysics, Fukuoka 807-0833 (JP)
(72) Inventor: MASAOKA, Katsunori, Hiroshima-shi Hiroshima 730-8652 (JP); MAEHARA, Kazuo, Hiroshima-shi Hiroshima 730-8652 (JP); CHIBA, Shingo, Hiroshima-shi Hiroshima 730-8652 (JP); KIM, Sung-Teh, Kitakyushu-shi Fukuoka 807-0831 (JP); YAMAMOTO, Chieko, Kitakyushu-shi Fukuoka 807-0833 (JP)
(74) Representative: Vossius & Partner
(86) International application number: PCT/JP2008/070955
(87) International publication number: WO 2009/069511

(57) **Abstract**

Provided is a hemodialysis apparatus which can fundamentally solve a problem in that air is trapped in a mesh disposed in a vein side chamber during priming. A hemodialysis apparatus includes control means that conducts, in the stated order: (A) a process in which a blood pump reversely rotates at the same speed as a reverse filtration speed made by third fluid feeding means, and a dialysate flows in a flow passage extending from a connection portion between a hemodialyzer and an artery side blood line to a vein side chamber through the joint portion in a loop formed by connecting the artery side blood line and the vein side blood line, to thereby prime the flow passage and the hemodialyzer, and (B) a process in which a reverse rotation speed of the blood pump is made lower than the reverse filtration speed made by the third fluid feeding means, and the dialysate of a flow rate corresponding to a speed obtained by subtracting the reverse rotation speed of the blood pump from the reverse filtration speed made by the third fluid feeding means flows in a flow passage extending from a connection portion between the hemodialyzer and the vein side blood line to the vein side chamber, which is a remaining flow passage of the loop, to thereby prime the flow passage and the hemodialyzer.

## Description

### Technical Field

The present invention relates to a hemodialysis apparatus that is used for a medical treatment with an extracorporeal circulation of blood such as hemodialysis, hemodialysis filtration, or hemofiltration, and more particularly to a hemodialysis apparatus that can fundamentally solve such a problem that air is trapped in a mesh disposed in a vein side chamber C_{V} during priming, and then remains therein.

### Background Art

A hemodialysis apparatus is a type of medical equipment that extracorporeally circulates blood of a patient with renal failure or a drug intoxicated patient to perform blood purification. The hemodialysis apparatus generally includes three portions of (1) a hemodialyzer D that brings blood into contact with a dialysate through a semipermeable membrane to purify blood, (2) a dialysate supply/discharge system mainly including a dialysate supply line L1 that supplies the dialysate to the hemodialyzer D, and a dialysate discharge line L2 that discharges the dialysate from the hemodialyzer D, and (3) a blood circuit mainly including an artery side blood line L3 that allows the blood drawn from the patient to flow into the hemodialyzer D, and a vein side blood line L4 that returns the blood drained from the hemodialyzer D to the patient.

In conducting the medical treatment using the above-mentioned hemodialysis apparatus, priming for washing the flow passages of the hemodialyzer D and a blood circuit including the artery side blood line L3, and the vein side blood line L4 by the aid of a normal saline or a dialysate is conducted as a preliminary process.

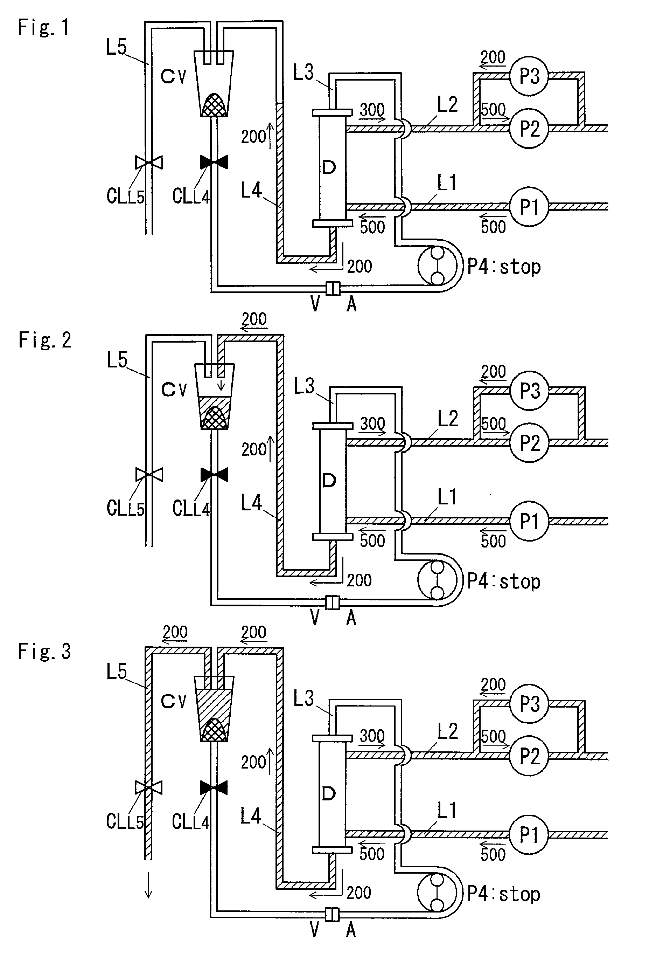

FIGS. 1 to 6 are diagrams illustrating an example of a priming method according to the latter conventional art using the dialysate. In this method, an extracorporeal circulation circuit is washed through three processes (for example, refer to Patent Document 1). Numerical numbers illustrated in the figures indicate flow rates of the dialysate, and arrows indicate directions along which the dialysate flows. A case in which the hemodialyzer D of the wet type is used is described below.

### (First Process)

In a first process , as illustrated in FIG. 1, a reverse filtering operation is conducted by third fluid feeding means P3 in a state where a blood pump P4 stops, and a clamp CL_{L4} disposed on a downstream side of the vein side chamber C_{V} is closed. For example, in a state where each of first fluid feeding means P1 and second fluid feeding means P2 is operated at a flow rate of 500 ml/min, the third fluid feeding means P3 is operated at 200 ml/min in the reverse filtration direction. With this operation, the flow rate of the dialysate pushed into the hemodialyzer D is larger than the flow rate pulled therefrom by 200 ml/min. Therefore, a reverse filtration phenomenon that the dialysate flowing outside a hollow fiber is pushed into the hollow fiber due to a difference in the flow rate occurs within the hemodialyzer D.

Then, the dialysate pushed into the hollow fiber within the hemodialyzer D through the reverse filtering operation due to the third fluid feeding means P3 flows, as illustrated in FIG. 1, in a flow passage extending from a connection portion between the hemodialyzer D and the vein side blood line L4 to the vein side chamber C_{V} in the stated direction at a rate of 200 ml/min, which is the same as the flow rate of the reverse filtering because the blood pump P4 stops. At the same time, the dialysate pushes out air in the flow passage, thus finally completing air removal in the flow passage as illustrated in FIG. 2.

Then, the dialysate that has flown in the vein side chamber C_{V} accumulates in the vein side chamber C_{V} illustrated in FIG. 2 because the clamp CL_{L4} disposed on the downstream side of the vein side chamber C_{V} is closed, and the dialysate that has reached a given height and has nowhere to go is discharged from an overflow line L5 as illustrated in FIG. 3.

As described above, in the first process, the dialysate that has been pushed into the hollow fiber within the hemodialyzer D through the reverse filtering operation due to the third fluid feeding means P3 flows in the flow passage extending from the connection portion between the hemodialyzer D and the vein side blood line L4 to the vein side chamber C_{V} in the stated direction to prime the flow passage and the hemodialyzer D.

### (Second Process)

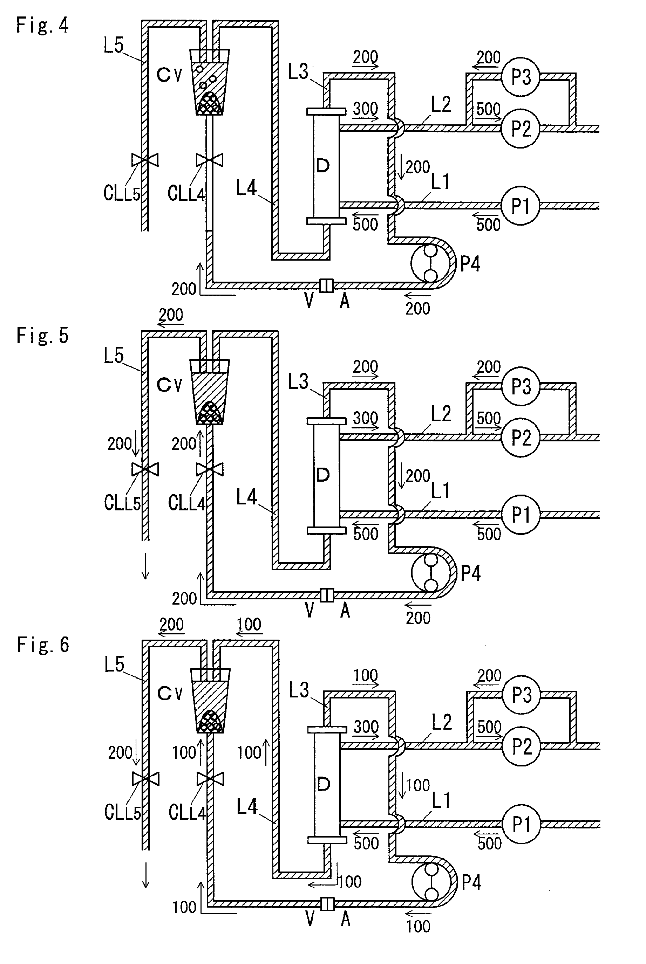

In a second process, as illustrated in FIG. 4, the blood pump P4 that has stopped reversely rotates at the same speed as that of a reverse filtration speed made by the third fluid feeding means P3, and the clamp CL_{L4} that has been closed is opened. As a result, the dialysate is not allowed to flow in the flow passage extending from the connection portion between the hemodialyzer D and the vein side blood line L4 to the vein side chamber C_{V}**,** and, as illustrated in FIG. 4, the dialysate that has been pushed into the hollow fiber within the hemodialyzer D through the reverse filtering operation due to the third fluid feeding means P3 flows in a flow passage extending from a connection portion between the hemodialyzer D and the artery side blood line L3 to the vein side chamber C_{V} through the blood pump P4 in the stated direction at 200 ml/min which is the same rate as the flow rate of the reverse filtration. At the same time, the dialysate pushes out air in the flow passage, thus finally completing air removal in the flow passage as illustrated in FIG. 5.

Then, because the dialysate accumulates in the vein side chamber C_{V} by priming made in the first process, the dialysate that has flown in the vein side chamber C_{V} is discharged from the overflow line L5 as illustrated in FIG. 5.

As described above, in the second process, the dialysate that has been pushed into the hollow fiber within the hemodialyzer D through the reverse filtering operation due to the third fluid feeding means P3 flows in the flow passage extending from the connection portion between the hemodialyzer D and the artery side blood line L3 to the vein side chamber C_{V} through the blood pump P4 in the stated direction to prime the flow passage and the hemodialyzer D.

### (Third Process)

In a third process, as illustrated in FIG. 6, the reverse rotation speed of the blood pump P4 is made lower than the reverse filtration speed made by the third fluid feeding means P3. For example, the blood pump P4 reversely rotates at 100 ml/min while the reverse filtration speed of the third fluid feeding means P3 is 200 ml/min. With this operation, the flow rate of the dialysate that has flown in the flow passage extending from the connection portion between the hemodialyzer D and the artery side blood line L3 to the vein side chamber C_{V} through the blood pump P4 in the stated direction in the second process decreases from 200 ml/min to 100 ml/min. On the other hand, the dialysate flows at the flow rate of 100 ml/min in the flow passage extending from the connection portion between the hemodialyzer D and the vein side blood line L4 to the vein side chamber C_{V} in the stated direction.

As described above, in the third process, the dialysate that has been pushed into the hollow fiber within the hemodialyzer D through the reverse filtering operation due to the third fluid feeding means P3 flows both in the flow passage extending from the connection portion between the hemodialyzer D and the artery side blood line L3 to the vein side chamber C_{V} through the blood pump P4 and in the flow passage extending from the connection portion between the hemodialyzer D and the vein side blood line L4 to the vein side chamber C_{V}, so as to prime the both flow passages and the hemodialyzer D. The general operation of the priming method according to the conventional art using the dialysate is described above.

Incidentally, the mesh disposed in the vein side chamber C_{V} is made of a hydrophobic material, and hence if the mesh is once wetted with the dialysate, air may hardly pass through the mesh due to surface tension. For that reason, in the above-mentioned priming method, after the mesh disposed in the vein side chamber C_{V} is wetted in the first process illustrated in FIGS. 1 to 3, the air in the flow passage extending from the connection portion between the hemodialyzer D and the artery side blood line L3 to the vein side chamber C_{V} through the blood pump P4 is allowed to pass through the wetted mesh as illustrated in FIG. 4. Therefore, there arises a problem in that the air is trapped in the mesh as illustrated in FIG. 4, and remains in the extracorporeal circulation circuit as illustrated in FIGS. 5 and 6.

If the air remains in the extracorporeal circulation circuit, there is a risk of air entrainment into a body of the patient during a medical treatment with an extracorporeal circulation of blood such as hemodialysis, hemodialysis filtration, or hemofiltration. For that reason, in the priming method according to the conventional art, in the processes illustrated in FIGS. 5 and 6, a chucking (flashing) operation for intermittently opening and closing the clamp CL_{L4} disposed on the downstream side of the vein side chamber C_{V} is conducted to remove the air that accumulates in the mesh disposed in the vein side chamber C_{V} (for example, refer to Patent Document 2).

However, from the viewpoint of providing a safe medical treatment with no accident to the patient, it is desirable to fundamentally solve the phenomenon in which the air is trapped in the mesh disposed in the vein side chamber C_{V} rather than a coping process of removing the trapped air ex-post facto assuming that the air is trapped in the mesh disposed in the vein side chamber C_{V}.
Patent Document 1: JP 2004-16619 A (paragraphs [0037] to [0039])
Patent Document 2: JP 2004-187990 A (paragraph [0051])

### Disclosure of the Invention

### Problem to be solved by the Invention

A problem to be solved by the present invention is to fundamentally solve a problem in that air is trapped in a mesh disposed in a vein side chamber C_{V} during priming, and remains therein, and to provide a hemodialysis apparatus which can prevent beforehand any medical accident caused by air entrainment during a medical treatment with an extracorporeal circulation of blood such as hemodialysis, hemodialysis filtration, or hemofiltration.

### Means for solving the Problem

The inventor of the present invention has attained a hemodialysis apparatus that can fundamentally solve the above-mentioned problem as a result of repeating various experimental studies and logical studies for solving the above-mentioned problem. The summary is described below.

(1) A hemodialysis apparatus, including: a hemodialyzer (D) of a wet type; a dialysate supply line (L1) that supplies a dialysate to the hemodialyzer (D); a dialysate discharge line (L2) that discharges the dialysate from the hemodialyzer (D); an artery side blood line (L3) that allows blood drawn from a patient to flow into the hemodialyzer (D); a vein side blood line (L4) that returns the blood drained from the hemodialyzer (D) to the patient; first fluid feeding means (P1) disposed in the dialysate supply line (L1); second fluid feeding means (P2) disposed in the dialysate discharge line (L2); third fluid feeding means (P3) that can rotate reversibly and is disposed in a bypass line that communicates an upstream side and a downstream side of any one or both of the first fluid feeding means (P1) and the second fluid feeding means (P2) with each other; a blood pump (P4) disposed in the artery side blood line (L3); a vein side chamber (C_{V}) including a mesh, disposed in the vein side blood line (L4); an overflow line (L5) connected to the vein side chamber (C_{V}); and control means (G1) that conducts, in the stated order: (A) a process in which the blood pump (P4) reversely rotates at the same speed as a reverse filtration speed made by the third fluid feeding means (P3), and the dialysate that has been pushed into a hollow fiber within the hemodialyzer (D) through a reverse filtering operation made by the third fluid feeding means (P3) flows in a first flow passage extending from a connection portion between the hemodialyzer (D) and the artery side blood line (L3) to the vein side chamber (C_{V}) through the joint portion in a loop formed by connecting the artery side blood line (L3) and the vein side blood line (L4), to thereby prime the first flow passage and the hemodialyzer (D); and (B) a process in which a reverse rotation speed of the blood pump (P4) is made lower than the reverse filtration speed made by the third fluid feeding means (P3), and the dialysate of a flow rate corresponding to a speed obtained by subtracting the reverse rotation speed of the blood pump (P4) from the reverse filtration speed made by the third fluid feeding means (P3) flows in a second flow passage extending from a connection portion between the hemodialyzer (D) and the vein side blood line (L4) to the vein side chamber (C_{V}), which is a remaining flow passage of the loop, to thereby prime the second flow passage and the hemodialyzer (D).

(2) A hemodialysis apparatus, including: a hemodialyzer (D) of a wet type or a dry type; a dialysate supply line (L1) that supplies a dialysate to the hemodialyzer (D); a dialysate discharge line (L2) that discharges the dialysate from the hemodialyzer (D); an artery side blood line (L3) that allows blood drawn from a patient to flow into the hemodialyzer (D); a vein side blood line (L4) that returns the blood drained from the hemodialyzer (D) to the patient; first fluid feeding means (P1) disposed in the dialysate supply line (L1); second fluid feeding means (P2) disposed in the dialysate discharge line (L2); third fluid feeding means (P3) that can rotate reversibly and is disposed in a bypass line that communicates an upstream side and a downstream side of any one or both of the first fluid feeding means (P1) and the second fluid feeding means (P2) with each other; a blood pump (P4) disposed in the artery side blood line (L3); a vein side chamber (C_{V}) including a mesh, disposed in the vein side blood line (L4); an overflow line (L5) connected to the vein side chamber (C_{V}); and control means (G2) that conducts a process in which the blood pump (P4) forwardly rotates at a speed lower than a reverse filtration speed made by the third fluid feeding means (P3), and the dialysate that has been pushed into a hollow fiber within the hemodialyzer (D) through a reverse filtering operation made by the third fluid feeding means (P3) circulates in a loop formed by connecting the artery side blood line (L3) and the vein side blood line (L4), which extends from a connection portion between the hemodialyzer (D) and the vein side blood line (L4) to a connection portion between the hemodialyzer (D) and the artery side blood line (L3) through the vein side chamber (C_{V}), to thereby prime the flow passages and the hemodialyzer (D).

### Effects of the Invention

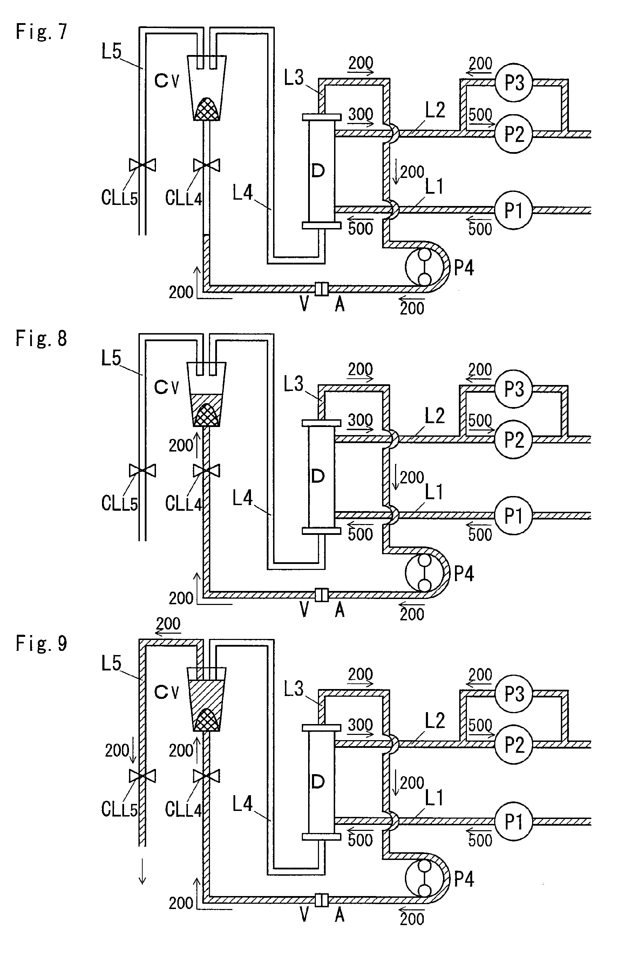

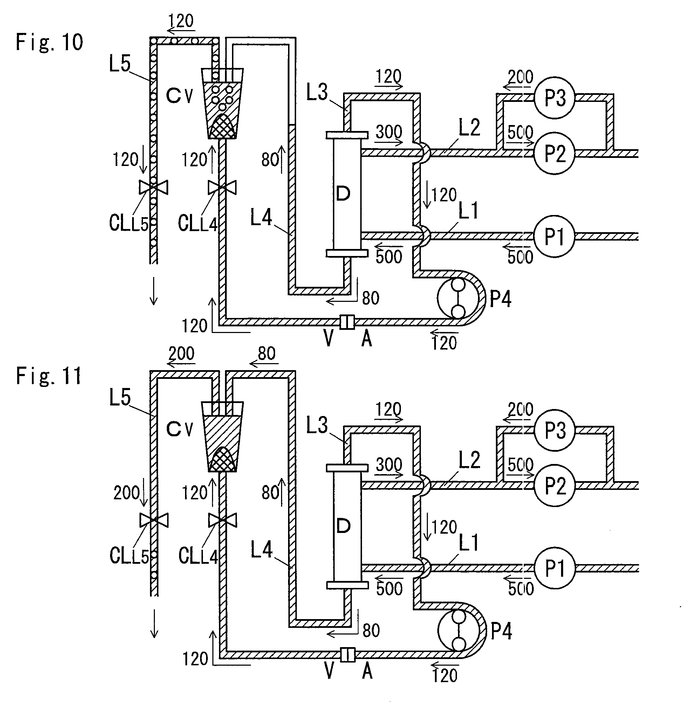

(1) According to the hemodialysis apparatus including the control means G1 according to the present invention, as the first process as illustrated in FIGS. 7 to 9, the dialysate that has been pushed into the hollow fiber within the hemodialyzer D through the reverse filtering operation due to the third fluid feeding means P3 is allowed to flow in the first flow passage extending from the connection portion between the hemodialyzer D and the artery side blood line L3 to the vein side chamber C_{V} through the joint portion in the loop formed by connecting the artery side blood line L3 and the vein side blood line L4 in the stated direction, to thereby prime the flow passage and the hemodialyzer D.
In this example, because the air within the first flow passage passes through the mesh disposed in the vein side chamber C_{V} earlier than the dialysate, the air is not trapped in the mesh.
Then, as illustrated in FIGS. 10 and 11, as the second process, the dialysate is allowed to flow in the second passage extending from the connection portion between the hemodialyzer D and the vein side blood line L4 to the vein side chamber C_{V}, which is the remaining flow passage of the loop, in the stated direction, to thereby prime the flow passage and the hemodialyzer D.
As illustrated in FIG. 10, the air within the second passage flows into the vein side chamber C_{V} earlier than the dialysate. However, the air that flows into the vein side chamber C_{V} does not reach the mesh disposed in the vein side chamber C_{V} due to a buoyancy of the dialysate that has accumulated in the vein side chamber C_{V} and an upward flow of the dialysate that flows in the first flow passage. Accordingly, the air is not trapped in the mesh.
That is, the hemodialysis apparatus including the control means G1 which conducts the first process and the second process in the stated order according to the present invention can fundamentally solve a problem in that the air is trapped in the mesh disposed in the vein side chamber C_{V} during priming, and remains therein, thereby enabling the medical accident caused by the air entrainment during the medical treatment to be prevented beforehand.

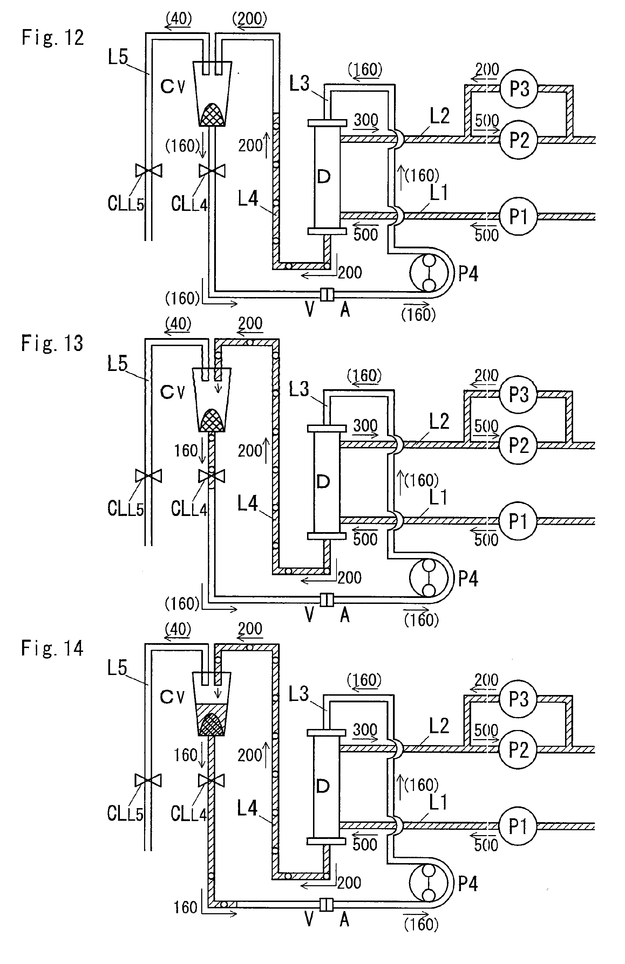

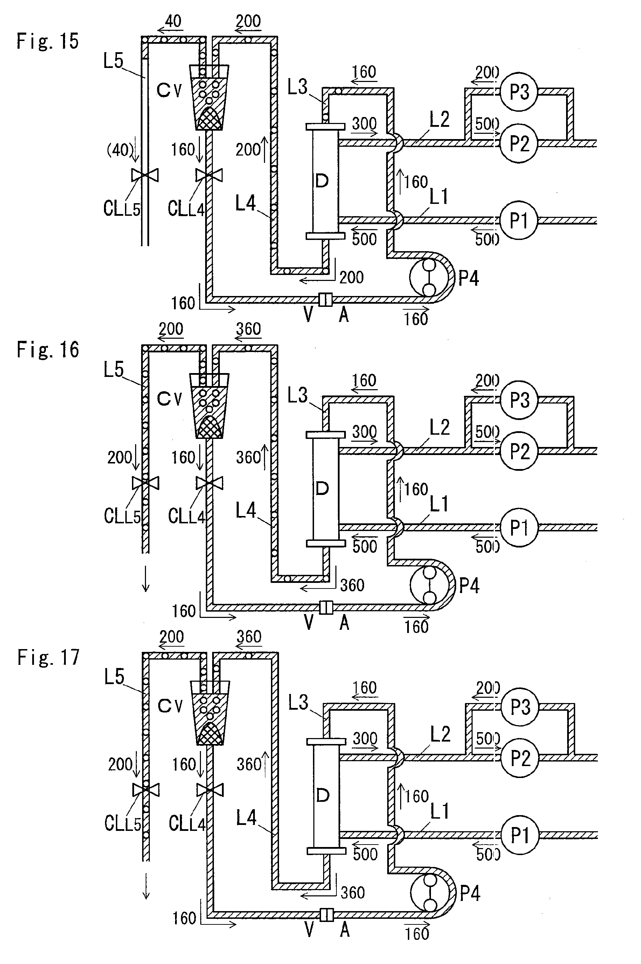

(2) According to the hemodialysis apparatus including the control means G2 according to the present invention, as illustrated in FIGS. 12 to 17, the dialysate that has been pushed into the hollow fiber within the hemodialyzer D through the reverse filtering operation due to the third fluid feeding means P3 is circulated in the loop formed by connecting the artery side blood line L3 and the vein side blood line L4, which extends from the connection portion between the hemodialyzer D and the vein side blood line L4 to the connection portion between the hemodialyzer D and the artery side blood line L3 through the vein side chamber C_{V}, in the stated direction, to thereby prime the flow passage and the hemodialyzer D. FIGS. 12 to 17 illustrate the dialysate and the air flow in time series in the case where the hemodialyzer D of a dry type is used.
As described above, if the air is allowed to pass through the mesh disposed in the vein side chamber C_{V} after the mesh is wetted with the dialysate, the air is trapped in the mesh. However, the mesh is wetted with the dialysate for the first time in a phase of FIG. 13. The air that flows into the mesh after the mesh has been wetted is discharged from the overflow line L5, and the air that has been trapped in the mesh by wetting the mesh is pulled into the vein side blood line L4 on the downstream side of the vein side chamber C_{V} together with the dialysate. Therefore, the above-mentioned air is not trapped in the mesh.
In a phase where the air that has been trapped in the mesh passes through the joint portion of the vein side blood line L4 and the artery side blood line L3, the blood pump P4, and the artery side blood line L3, and flows into the hemodialyzer D, the dialysate of a sufficient amount to be discharged from the overflow line L5 accumulates in the vein side chamber C_{V}. For that reason, the air that flows into the vein side chamber C_{V} does not reach the mesh disposed in the vein side chamber C_{V} due to the buoyancy of the dialysate that has accumulated in the vein side chamber C_{V} as illustrated in FIG. 15. Accordingly, the air is not trapped in the mesh.
That is , the hemodialysis apparatus including the control means G2 according to the present invention can fundamentally solve a problem in that the air is trapped in the mesh disposed in the vein side chamber C_{V} during priming, and remains therein, even when the hemodialyzer D of the dry type is used, thereby enabling the medical accident caused by air entrainment during the medical treatment to be prevented beforehand.

### Brief description of the Drawings

FIG. 1 is a schematic diagram for describing a priming method according to a conventional art.
FIG. 2 is a schematic diagram for describing the priming method according to the conventional art.
FIG. 3 is a schematic diagram for describing the priming method according to the conventional art.
FIG. 4 is a schematic diagram for describing the priming method according to the conventional art.
FIG. 5 is a schematic diagram for describing the priming method according to the conventional art.
FIG. 6 is a schematic diagram for describing the priming method according to the conventional art.
FIG. 7 is a schematic diagram for describing an operation of a hemodialysis apparatus according to a first embodiment of the present invention.
FIG. 8 is a schematic diagram for describing the operation of the hemodialysis apparatus according to the first embodiment of the present invention.
FIG. 9 is a schematic diagram for describing the operation of the hemodialysis apparatus according to the first embodiment of the present invention.
FIG. 10 is a schematic diagram for describing the operation of the hemodialysis apparatus according to the first embodiment of the present invention.
FIG. 11 is a schematic diagram for describing the operation of the hemodialysis apparatus according to the first embodiment of the present invention.
FIG. 12 is a schematic diagram for describing an operation of a hemodialysis apparatus according to a second embodiment of the present invention.
FIG. 13 is a schematic diagram for describing the operation of the hemodialysis apparatus according to the second embodiment of the present invention.
FIG. 14 is a schematic diagram for describing the operation of the hemodialysis apparatus according to the second embodiment of the present invention.
FIG. 15 is a schematic diagram for describing the operation of the hemodialysis apparatus according to the second embodiment of the present invention.
FIG. 16 is a schematic diagram for describing the operation of the hemodialysis apparatus according to the second embodiment of the present invention.
FIG. 17 is a schematic diagram for describing the operation of the hemodialysis apparatus according to the second embodiment of the present invention.

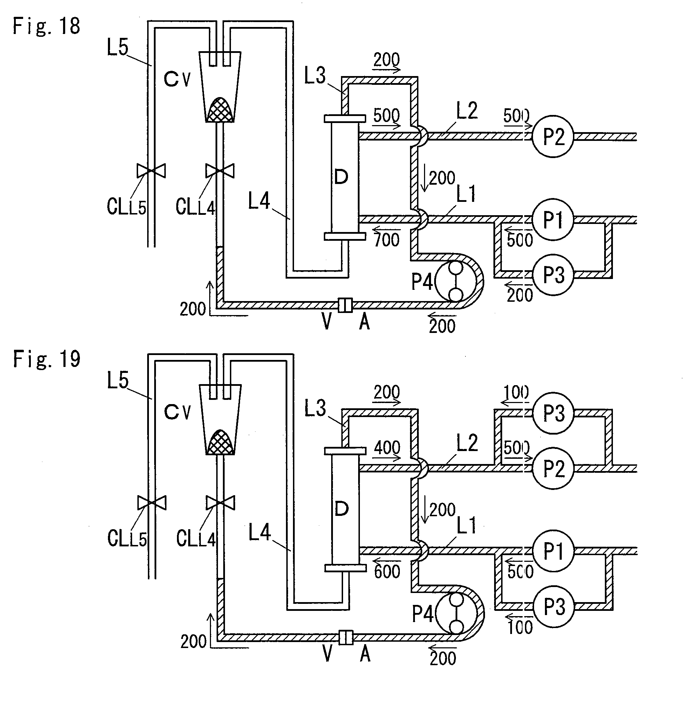

FIG. 18 is a schematic diagram illustrating one exemplary arrangement of third fluid feeding means P3.
FIG. 19 is a schematic diagram illustrating another exemplary arrangement of the third fluid feeding means P3.

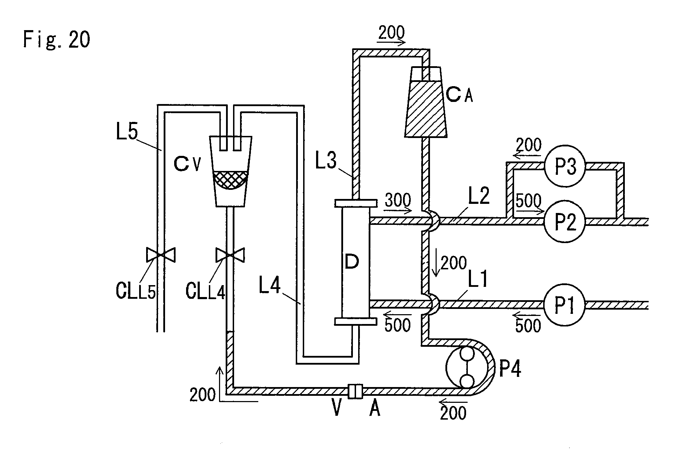

FIG. 20 is a schematic diagram illustrating a hemodialysis apparatus according to another embodiment of the present invention.

Description of Symbols
- C_{A}:: artery side chamber
- C_{V}:: vein side chamber
- CL_{L4}:: clamp disposed on downstream side of vein side chamber C_{V}
- CL_{L5}:: clamp disposed in overflow line L5
- D:: hemodialyzer
- L1:: dialysate supply line
- L2:: dialysate discharge line
- L3:: artery side blood line
- L4:: vein side blood line
- L5:: overflow line
- P1:: first fluid feeding means
- P2:: second fluid feeding means
- P3:: third fluid feeding means
- P4:: blood pump

### Best mode for carrying out the Invention

First, a hemodialysis apparatus according to a first embodiment of the present invention is described. Hereinafter, the hemodialysis apparatus according to the first embodiment of the present invention is referred to as "first embodiment".
The first embodiment is a hemodialysis apparatus including a hemodialyzer D of a wet type.
FIGS. 7 to 11 are diagrams illustrating the operation of the first embodiment, and flows of a dialysate and air due to the operation. Numerical values illustrated in the figures indicate flow rates of the dialysate, and arrows indicate directions along which the dialysate flows.
The first embodiment is characterized in flowpassage selection and a washing direction of the priming solution (dialysate) due to control means G1 which is described.

### (First Process)

In a first process, as illustrated in FIG. 7, a blood pump P4 reversely rotates at the same speed as a reverse filtration speed made by third fluid feeding means P3. For example, in a state where each of first fluid feeding means P1 and second fluid feeding means P2 is operated at a flow rate of 500 ml/min, the third fluid feeding means P3 is operated at 200 ml/min in the reverse filtration direction, and the blood pump P4 reversely rotates at 200 ml/min that is the same speed as the reverse filtration speed made by the third fluid feeding means P3. With this operation, the flow rate of the dialysate pushed into the hemodialyzer D is larger than the flow rate pulled therefrom by 200 ml/min. Therefore, a reverse filtration phenomenon that the dialysate that has flown outside a hollow fiber is pushed into the hollow fiber due to a difference in the flow rate occurs within the hemodialyzer D.
The reverse rotation means rotation in a direction opposite to a direction (forward rotation direction) along which the blood pump P rotates during a dialysis treatment.

The dialysate that has been pushed into the hollow fiber within the hemodialyzer D through the reverse filtering operation made by the third fluid feeding means P3 flows in a first flow passage extending from a connection portion between the hemodialyzer D and an artery side blood line L3 to a vein side chamber C_{V} through a junction portion in a loop formed by connecting the artery side blood line L3 and a vein side blood line L4 in the stated direction at 200 ml/min which is the same rate as the flow rate of the reverse filtration as illustrated in FIG. 7, because the blood pump P4 reversely rotates at the same speed as the reverse filtration speed made by the third fluid feeding means P3. At the same time, the dialysate pushes out the air in the flow passage, thus finally completing air removal in the flow passage as illustrated in FIG. 8.

In this example, because the air within the first flow passage passes through a mesh disposed in the vein side chamber C_{V} earlier than the dialysate, the air is not trapped in the mesh. That is, because the mesh disposed in the vein side chamber C_{V} is made of a hydrophobic material, if the air passes through the mesh wetted with the dialysate, the air is trapped in the mesh by surface tension action. However, in this process, as illustrated in FIGS. 7 and 8, because the mesh is wetted with the dialysate after all the air within the first flow passage has passed through the mesh, the air within the first flow passage is not trapped in the wetted mesh.

Then, the dialysate that has flown into the vein side chamber C_{V} accumulates in the vein side chamber C_{V} as illustrated in FIG. 8, and the dialysate that reached a given height and has nowhere to go is discharged from an overflow line L5 as illustrated in FIG. 9.

As described above, in the first process, the blood pump P4 reversely rotates at the same speed as the reverse filtration speed made by the third fluid feeding means P3 so that the dialysate that has been pushed into the hollow fiber within the hemodialyzer D through the reverse filtering operation made by the third fluid feedingmeans P3 flows in the first flowpassage in the above-mentioned direction, to thereby prime the flow passage and the hemodialyzer D.

When the blood pump P4 reversely rotates at a speed higher than the reverse filtration speed made by the third fluid feeding means P3, the flow rate of the dialysate that flows in the first flow passage is the same as that when the blood pump P4 reversely rotates at the same speed as the reverse filtration speed made by the third fluid feeding means P3. However, the air is pulled into the hemodialyzer D from the vein side blood line L4, which is undesirable.
On the other hand, when the blood pump P4 reversely rotates at a speed lower than the reverse filtration speed made by the third fluid feeding means P3, the dialysate also flows in the second flow passage extending from the connection portion between the hemodialyzer D and the vein side blood line L4 to the vein side chamber C_{V} in the stated direction. For example, when the reverse rotation speed of the blood pump P4 is set to 100 ml/min in FIG. 7, the dialysate flows into both of the first flow passage and the second flow passage at the flow rate of 100 ml/min. Accordingly, there may occur a situation in which the mesh disposed in the vein side chamber C_{V} is wetted with the dialysate that has flown from the second flow passage before the removal of the air from the first flow passage has been completed although depending on a volume ratio of the first flow passage and the second flow passage.
That is, it is most desirable that the reverse rotation speed of the blood pump P4 be the same as the reverse filtration speed made by the third fluid feeding means P3. However, the speed lower than the reverse filtration speed made by the third fluid feeding means P3 is not completely excluded. Even if the speed is lower than the reverse filtration speed made by the third fluid feeding means P3, when such a speed is a speed at which the dialysate from the second flow passage flows into the vein side chamber C_{V} after the removal of the air from the first flow passage has been completed, that is, after all of the air from the first flow passage has passed through the mesh disposed in the vein side chamber C_{V}, the object of the first embodiment can be achieved.

### (Second Process)

In a second process, as illustrated in FIG. 10, the reverse rotation speed of the blood pump P4 is made lower than the reverse filtration speed made by the third fluid feeding means P3. For example, the blood pump P4 reversely rotates at 120 ml/min while the reverse filtration speed made by the third fluid feeding means P3 is 200 ml/min. With this operation, the flow rate of the dialysate that has flown in the first flow passage in the first process decreases from 200 ml/min to 120 ml/min. On the other hand, the dialysate flows in the second flow passage extending from the connection portion between the hemodialyzer D and the vein side blood line L4 to the vein side chamber C_{V} in the stated direction at the flow rate of 80 ml/min, which is a flow rate corresponding to a speed obtained by subtracting the reverse rotation speed of the blood pump P4 from the reverse filtration speed made by the third fluid feeding means P3, and also pushes the air out of the flow passage.

Then, because the dialysate has accumulated in the vein side chamber C_{V} by priming in the first process, the air and the dialysate that flow into the vein side chamber C_{V} are discharged from the overflow line L5 as illustrated in FIG. 10, thereby finally completing the air removal from the second flow passage as illustrated in FIG. 11.

The air within the second flow passage flows into the vein side chamber C_{V} earlier than the dialysate as illustrated in FIG. 10, but the air that flows into the vein side chamber C_{V} does not reach the mesh disposed in the vein side chamber C_{V} due to a buoyancy of the dialysate that has accumulated in the vein side chamber C_{V} and an upward flow of the dialysate that flows in the first flow passage as illustrated in FIG. 10. Accordingly, the air is not trapped in the mesh.

As described above, in the second passage, the dialysate that has been pushed into the hollow fiber within the hemodialyzer D through the reverse filtering operation made by the third fluid feeding means P3 flows into both of the first flow passage and the second flow passage, to thereby prime those flow passages and the hemodialyzer D.

The general operation of the first embodiment including the control means G1 which conducts the first process and the second process in the stated order is described above. According to the first embodiment, such a problem that the air is trapped in the mesh disposed in the vein side chamber C_{V} during priming, and remains therein, may be fundamentally solved, thereby enabling the medical accident caused by the air entrainment during the medical treatment to be prevented beforehand.

It should be noted that in the second process described above, as illustrated in FIG. 10, the reverse rotation speed of the blood pump P4 is made lower than the reverse filtration speed made by the third fluid feeding means P3 so that the dialysate that has been pushed into the hollow fiber within the hemodialyzer D through the reverse filtering operation made by the third fluid feeding means P3 flows into both of the first flow passage and the second flow passage to thereby prime those flow passages and the hemodialyzer D. Alternatively, the blood pump P4 may stop so that the dialysate flows only in the second flow passage, thereby allowing the above-mentioned flow passage and the hemodialyzer D to be primed.
However, in this case, because the air that flows into the vein side chamber C_{V} from the second flow passage cannot obtain the upward flow of the dialysate that flows in the first flow passage, the air that flows into the vein side chamber C_{V} reaches a deeper portion of the vein side chamber C_{V} than that when the dialysate flows in both of the first flow passage and the second flow passage. For that reason, when the blood pump P4 stops so that the dialysate flows only in the second flow passage, the reverse filtration speed made by the third fluid feeding means P3 should be decreased from the viewpoint of decreasing the inflow speed of the air that flows into the vein side chamber C_{V} from the second flow passage.

As to a transition moment from the first process to the second process, it is desirable to transition to the second process at a moment when the dialysate that has washed the first flow passage in the first process as illustrated in FIG. 9 is discharged from the overflow line L5 because the first flow passage is washed also in the second process.
Further, when the dialysate from the second flow passage flows into the vein side chamber C_{V} after all of the air within the first flow passage has passed through the mesh disposed in the vein side chamber C_{V}, the air within the first flow passage is not trapped in the wetted mesh. Therefore, it is possible to transition to the second process at a moment when the dialysate flows into the vein side chamber C_{V} from the first flow passage.

Alternatively, the amount of dialysate necessary to prime the hemodialysis apparatus, that is, the amount of dialysate necessary to wash the flow passages of the hemodialyzer D and the blood circuit including the artery side blood line L3 and the vein side blood line L4 is managed according to the reverse filtration speed and the period of time made by the third fluid feeding means P3, and that information is saved in given recording means such as a memory or a hard disk. The hemodialysis apparatus automatically starts priming according to priming start information from a healthcare professional, for example, upon pressing a priming start button disposed in the hemodialysis apparatus, and operates the third fluid feeding means P3 based on the reverse filtration speed and the period of time which have been saved in the recording means. Accordingly, from the viewpoint of controllability, it is possible that a period of time when the dialysate that has washed the first flow passage is discharged from the overflow line L5, or a period of time when the dialysate flows into the vein side chamber C_{V} from the first flow passage is calculated in advance, the calculated period of time is saved in given recording means such as a memory or a hard disk in advance, and the transition to the second process is performed after the calculated period of time has elapsed from a time point when the priming start button has been pressed. With application of this method, an intervention of the healthcare professional is not required, and the transition to the second process may be automatically performed.

Subsequently, a hemodialysis apparatus according to a second embodiment of the present invention is described. Hereinafter, the hemodialysis apparatus according to the second embodiment of the present invention is referred to as "second embodiment".
The second embodiment is a hemodialysis apparatus including a hemodialyzer D of the wet type or the dry type.
FIGS. 12 to 17 are diagrams illustrating the operation of the second embodiment when the hemodialyzer D of the dry type is used and flows of a dialysate and the air due to the operation. Numerical values illustrated in the figures indicate flow rates of the dialysate, and arrows indicate directions along which the dialysate flows. The numerical values in the figures each indicate a flow rate of the dialysate including air when the air exists in a flow passage located at the number.
The second embodiment is also characterized in the flow selection and the washing direction of the priming solution due to the control means G2, which is described.

According to the second embodiment, as illustrated in FIGS. 12 to 17, the blood pump P4 forwardly rotates at a speed lower than the reverse filtration speed made by the third fluid feeding means P3. For example, in a state where each of the first fluid feeding means P1 and the second fluid feeding means P2 is operated at a flow rate of 500 ml/min, the third fluid feeding means P3 is operated at 200 ml/min in the reverse filtration direction, and the blood pump P4 forwardly rotates at 160 ml/min. With this operation, the flow rate of the dialysate pushed into the hemodialyzer D is larger than the flow rate pulled therefrom by 200 ml/min. Therefore, a reverse filtration phenomenon that the dialysate that has flown outside the hollow fiber is pushed into the hollow fiber due to a difference in the flow rate occurs within the hemodialyzer D.

As illustrated in FIG. 12, the dialysate that has been pushed into the hollow fiber within the hemodialyzer D through the reverse filtering operation made by the third fluid feeding means P3 flows in the flow passage extending from the connection portion between the hemodialyzer D and the vein side blood line L4 to the vein side chamber C_{V} in a loop formed by connecting the artery side blood line L3 and the vein side blood line L4, in the stated direction at a flow rate of 200 ml/min. At the same time, the dialysate pushes the air out of the flow passage, thus finally completing the air removal from the flow passage as illustrated in FIG. 13. In a phase illustrated in FIG. 12, the blood pump P4 pulls the air from the vein side chamber C_{V} when the dialysate has not reached the vein side chamber C_{V} at 160 ml/min, and pushes the air into the hemodialyzer D, which is meant by (160) in the figure.

FIG. 13 illustrates a case in which the hemodialyzer D of the dry type is used. Therefore, completion of the air removal from the flow passage means that all of the air existing in the flow passage extending from the connection portion between the hemodialyzer D and the vein side blood line L4 to the vein side chamber C_{V} has been pushed out by the dialysate. The air to be pushed out of the hemodialyzer D of the dry type exists in the flow passage in this phase as illustrated in FIG. 13. Mark "o" illustrated in the flow passage means the air that has been pushed out of the hemodialyzer D of the dry type.
It should be noted that, a case, in which the hemodialyzer D of the wet type is used, is different from the above-mentioned case, and means that all of the air existing in the flow passage has been pushed out by the dialysate.

The reason that the dialysate flows in the flow passage at the flow rate of 200 ml/min although the blood pump P4 forwardly rotates at 160 ml/min is that the dialysate of 200 ml/min which has been pushed into the hollow fiber by the reverse filtration operation made by the third fluid feeding means P3 has nowhere to go other than the flow passage extending from the connection portion between the hemodialyzer D and the vein side blood line L4 to the vein side chamber C_{V} because the blood pump P4 forwardly rotates.

If the completion of the air removal from the flow passage, the dialysate that has been pushed into the hollow fiber within the hemodialyzer D through the reverse filtration operation made by the third fluid feeding means P3, and the air that has been pushed out of the hemodialyzer D of the dry type flow into the vein side chamber C_{V} as illustrated in FIG. 13.

FIG. 13 is a diagram illustrating a state immediately after the dialysate and the air that has been pushed out of the hemodialyzer D of the dry type have flown into the vein side chamber C_{V}. The air is discharged from the overflow line L5 at 40 ml/min. On the other hand, the dialysate drops into the vein side chamber C_{V}, and wets the mesh. At the same time, the dialysate is pulled into the vein side blood line L4 on the downstream side of the vein side chamber C_{V} at the flow rate of 160 ml/min together with the air that has been trapped in the mesh because the mesh has been wetted. This is because immediately after the dialysate and the air that has been pushed out of the hemodialyzer D of the dry type have first flown into the vein side chamber C_{V}, no dialysate righteously accumulates in the vein side chamber C_{V}, and the blood pump P4 that rotates forwardly pulls the dialysate at 160 ml/min. Further, the reason that the air is discharged from the overflow line L5 at 40 ml/min is because the dialysate and the air flow into the vein side chamber C_{V} at 200 ml/min while being pulled at 160 ml/min, and therefore 40 ml/min that is a difference therebetween is discharged from the overflow line L5.

That is, as described above, when the air passes through the mesh after the mesh disposed in the vein side chamber C_{V} has been wetted with the dialysate, the air is trapped in the mesh. However, the mesh is wetted with the dialysate for the first time in a phase of FIG. 13. As described above, the air that flows into the vein side chamber C_{V} after the mesh has been wetted therewith is discharged from the overflow line L5 . The air that has been trapped in the mesh because the mesh has been wetted, in a strict sense, the air that has originally existed in the vein side chamber C_{V} and trapped in the mesh by membranes of the dialysate formed on surfaces of the mesh because the mesh has been wetted is pulled into the vein side blood line L4 on the downstream side of the vein side chamber C_{V} together with the dialysate. The air is not trapped in the mesh.

The dialysate and the air that have been pulled into the vein side blood line L4 from the vein side chamber C_{V} by the forward rotate operation of the blood pump P4 are still pulled into the blood pump P4 at 160 ml/min as illustrated in FIG. 14 while the dialysate flows in the vein side chamber C_{V} at 200 ml/min. Therefore, the dialysate accumulates in the vein side chamber C_{V} at the flow rate of 40 ml/min which is a difference therebewteen. That is, that the dialysate accumulates therein means that the air that has originally existed in the vein side chamber C_{V} and trapped in the mesh by membranes of the dialysate formed on surfaces of the mesh because the mesh has been wetted has escaped from the membranes. Therefore, only the dialysate is then pulled from the vein side chamber C_{V}.

FIG. 15 is a diagram illustrating a state immediately before the air that has been trapped in the mesh passes through the joint portion of the vein side blood line L4 and the artery side blood line L3, the blood pump P4, and the artery side blood line L3, and flows into the hemodialyzer D. In this phase, the dialysate of a sufficient amount to be discharged from the overflow line L5 has accumulated in the vein side chamber C_{V}. Accordingly, the air that flows into the vein side chamber C_{V} goes up in the dialysate due to the buoyancy of the dialysate that has accumulated in the vein side chamber C_{V} as illustrated in FIG. 15, and does not reach the mesh disposed in the vein side chamber C_{V}. Accordingly, the air is not trapped in the mesh.
The air that flows in the vein side chamber C_{V} in this phase means the air that has originally existed in the hemodialyzer D of the dry type, and the air that has been pulled out of the vein side chamber C_{V} when the dialysate has not reached the vein side chamber C_{V}, and pushed into the hemodialyzer D by the blood pump P4. The air also includes air that has trapped in the mesh and then flown into the hemodialyzer D.

As described above, in the phase illustrated in FIG. 15, the dialysate of a sufficient amount to be discharged from the overflow line L5 has accumulated in the vein side chamber C_{V}. Accordingly, the dialysate and the air that flow into the vein side chamber C_{V} are discharged from the overflow line L5 at the flow rate of 40 ml/min.

FIG. 16 is a diagram illustrating a state after the air that has been trapped in the mesh has flown into the hemodialyzer D. In this phase, the flow rate of the dialysate and the air that flow in the flow passage extending from the connection portion between the hemodialyzer D and the vein side blood line L4 to the vein side chamber C_{V} is 360 ml/min. This is because the dialysate of 160 ml/min which has been returned after circulating the loop is added to the dialysate of 200 ml/min that has been pushed into the hollow fiber by the reverse filtering operation made by the third fluid feeding means P3.
Further, the dialysate and the air flow into the vein side chamber Cyan 360 ml/min while the dialysate is pulled out of the vein side chamber C_{V} at 160 ml/min. Therefore, the dialysate and the air are discharged from the overflow line L5 at 200 ml/min which is a difference therebewteen.

As described above, because the air that flows into the vein side chamber C_{V} is continuously discharged from the overflow line L5, the air is reduced every time the dialysate is circulated as illustrated in FIG. 17, and finally disappears. In other words, all of the air that had existed in the hemodialyzer D of the dry type is replaced with the dialysate. Further, all of the air that has been pulled out of the vein side chamber C_{V} when the dialysate has not reached the vein side chamber C_{V}, and pushed into the hemodialyzer D by the blood pump P4, or the air that has been trapped in the mesh because the mesh has been wetted, and pushed into the hemodialyzer D is discharged from the overflow line L5, and disappears from the loop formed by connecting the artery side blood line L3 and the vein side blood line L4, which extends from the connection portion between the hemodialyzer D and the vein side blood line L4 to the connection portion between the hemodialyzer D and the artery side blood line L3 through the vein side chamber C_{V}.

That is, the second embodiment forwardly rotates the blood pump P4 to circulate the dialysate that has been pushed into the hollow fiber within the hemodialyzer D through the reverse filtering operation made by the third fluid feeding means P3 in the loop in the stated direction, to thereby prime the flow passage and the hemodialyzer D.

The general operation of the second embodiment is described above. According to the second embodiment, needless to say the hemodialyzer D of the wet type, even when the hemodialyzer D of the dry type is used, such a problem that the air is trapped in the mesh disposed in the vein side chamber C_{V} during priming, and remains therein, may be fundamentally solved, thereby enabling the medical accident caused by the air entrainment during the medical treatment to be prevented beforehand.

As described above, in the second embodiment, the air that has flown into the vein side chamber C_{V} goes up in the dialysate due to the buoyancy of the dialysate that has accumulated in the vein side chamber C_{V} as illustrated in FIGS. 15 to 17, and therefore does not reach the mesh disposed in the vein side chamber C_{V}. Accordingly, the air is not trapped in the mesh.
However, in the second embodiment, because the upward flow of the dialysate cannot be obtained unlike the first embodiment, the air that has flown into the vein side chamber C_{V} reaches a deeper portion of the vein side chamber C_{V} than that in the first embodiment. That is, in the case of the first embodiment, because the blood pump p4 reversely rotates as illustrated in FIG. 10, the air that has flown in the dialysate accumulating in the vein side chamber C_{V} goes up in the dialysate due to the upward flow of the dialysate that is pushed from the lower side of the vein side chamber C_{V}, and caused to act in a direction that does not move closer to the mesh. On the contrary, in the second embodiment, because the dialysate is pulled from the lower side of the vein side chamber C_{V}, the air that has flown in the dialysate accumulating in the vein side chamber C_{V} is caused to act in a direction that moves closer to the mesh.
Accordingly, a performance that pulls the air from the lower side of the vein side chamber C_{V} is determined according to the flow rate of the blood pump P4. Therefore, it is desirable to determine the flow rate of the blood pump P4 so that the buoyancy of the dialysate that has accumulated in the vein side chamber C_{V} exceeds a pulling force from the lower side of the vein side chamber C_{V}, and the air that has flown into the vein side chamber C_{V} does not reach the mesh, taking the flow rate flowing into the dialysate that has accumulated in the vein side chamber C_{V}, the depth of the vein side chamber C_{V}, and the shape and arrangement of the mesh into consideration.

It is desirable that the hemodialyzer D that brings blood into contact with the dialysate through a semipermeable membrane to purify the blood be of a hollow fiber type.

It is desirable that the dialysate supply line L1 that supplies the dialysate to the hemodialyzer D and the dialysate discharge line L2 that discharges the dialysate from the hemodialyzer D each be formed of a silicon tube.
Further, it is desirable that the artery side blood line L3 that allows the blood drawn from the patient to flow into the hemodialyzer D and the vein side blood line L4 that returns the blood drained from the hemodialyzer D to the patient each be made of a flexible chemosynthetic material.

It is desirable that the first fluid feeding means P1 that feeds the dialysate to the hemodialyzer D, and the second fluid feeding means P2 that sucks the dialysate from the hemodialyzer D each be formed of a diaphragm pump or a duplex pump.
Further, it is desirable that the blood pump 4 that circulates the blood and the like be formed of a roller tubing pump.

It is desirable that the third fluid feeding means P3 that moves the dialysate into the blood circuit by reverse filtration through the hemodialyzer D, and removes the blood within the hemodialyzer D be formed of a reversible metering pump.
In the above-mentioned first and second embodiments, the third fluid feedingmeans P3 is disposed in the bypass line that communicates the upstream side and the downstream side of the second fluid feeding means with each other. However, the present invention is not limited to this configuration.
In FIG. 18, the third feeding means P3 is disposed in the bypass line that communicates the upstream side and the downstream side of the first fluid feeding means P1 with each other. In this case, the reverse filtration speed made by the third fluid feeding means P3 is 200 ml/min.
In FIG. 19, the third feeding means P3 is disposed in each of the bypass line that communicates the upstream side and the downstream side of the first fluid feeding means P1 with each other, and the bypass line that communicates the upstream side and the downstream side of the second fluid feeding means P2 with each other. In this case, the reverse filtration speed made by the third fluid feeding means P3 is 200 ml/min obtained by adding the reverse filtration speed 100 ml/min made by the third fluid feeding means P3 disposed on the first fluid feeding means P1 side and the reverse filtration speed 100 ml/min made by the third fluid feeding means P3 disposed on the second fluid feeding means P2 side together.

It is desirable that the vein side chamber C_{V} disposed in the vein side blood line L4 be made of a flexible chemosynthetic material.
Further, it is desirable that in the overflow line L5 connected to the vein side chamber C_{V} be made of a flexible chemosynthetic material.
The above-mentioned first and second embodiments each includes only the vein side chamber C_{V}. Alternatively, an artery side chamber C_{A} may be disposed as illustrated in FIG. 20.
Further, the shape and arrangement of the mesh disposed in the vein side chamber C_{V} are not limited to the embodiments in which a mesh convex in the upward direction is disposed on the lower portion of the vein side chamber C_{V} as illustrated in FIGS. 7 to 19, and, as illustrated in FIG. 20, a mesh convex in the downward direction may be disposed on a middle position of the vein side chamber C_{V}.

It is desirable that the control means G1 that conducts control for reversely rotating the blood pump P4 at the same speed as the reverse filtration speed of the third fluid feeding means P3 recorded in given recording means based on this reverse filtration speed upon inputting the priming start information from the healthcare professional in the first process of the first embodiment, control for automatically transitioning to the second process based on the reverse filtration time of the third fluid feeding means P3 recorded therein, and control for controlling the reverse rotation speed of the blood pump P4 to a predetermined speed lower than the reverse filtration speed made by the third fluid feeding means P3 to complete the priming after a recorded given period of time has elapsed, be formed of a computer (electronic computer).
Likewise, it is desirable that the control means G2 that conducts control for forwardly rotating the blood pump P4 at a speed lower than the reverse filtration speed of the third fluid feeding means P3, which is also a predetermined speed recorded in given recording means, upon inputting the priming start information from the healthcare professional in the second embodiment, and control for completing the priming after a predetermined given period of time has elapsed, be formed of a computer (electronic computer).

## Claims

1. A hemodialysis apparatus, comprising:
a hemodialyzer (D) of a wet type;
a dialysate supply line (L1) that supplies a dialysate to the hemodialyzer (D);
a dialysate discharge line (L2) that discharges the dialysate from the hemodialyzer (D);
an artery side blood line (L3) that allows blood drawn from a patient to flow into the hemodialyzer (D);
a vein side blood line (L4) that returns the blood drained from the hemodialyzer (D) to the patient;
first fluid feeding means (P1) disposed in the dialysate supply line (L1);
second fluid feeding means (P2) disposed in the dialysate discharge line (L2);
third fluid feeding means (P3) that can rotate reversibly and is disposed in a bypass line that communicates an upstream side and a downstream side of any one or both of the first fluid feeding means (P1) and the second fluid feeding means (P2) with each other;
a blood pump (P4) disposed in the artery side blood line (L3);
a vein side chamber (C_{V}) including a mesh, disposed in the vein side blood line (L4);
an overflow line (L5) connected to the vein side chamber (C_{V});
and
control means (G1) that conducts, in the stated order:
(A) a process in which the blood pump (P4) reversely rotates at the same speed as a reverse filtration speed made by the third fluid feeding means (P3), and the dialysate that has been pushed into a hollow fiber within the hemodialyzer (D) through a reverse filtering operation made by the third fluid feeding means (P3) flows in a first flow passage extending from a connection portion between the hemodialyzer (D) and the artery side blood line (L3) to the vein side chamber (C_{V}) through the joint portion in a loop formed by connecting the artery side blood line (L3) and the vein side blood line (L4), to thereby prime the first flow passage and the hemodialyzer (D); and
(B) a process in which a reverse rotation speed of the blood pump (P4) is made lower than the reverse filtration speed made by the third fluid feeding means (P3), and the dialysate of a flow rate corresponding to a speed obtained by subtracting the reverse rotation speed of the blood pump (P4) from the reverse filtration speed made by the third fluid feeding means (P3) flows in a second flow passage extending from a connection portion between the hemodialyzer (D) and the vein side blood line (L4) to the vein side chamber (C_{V}), which is a remaining flow passage of the loop, to thereby prime the second flow passage and the hemodialyzer (D).

2. A hemodialysis apparatus, comprising:
a hemodialyzer (D) of a wet type or a dry type;
a dialysate supply line (L1) that supplies a dialysate to the hemodialyzer (D);
a dialysate discharge line (L2) that discharges the dialysate from the hemodialyzer (D);
an artery side blood line (L3) that allows blood drawn from a patient to flow into the hemodialyzer (D);
a vein side blood line (L4) that returns the blood drained from the hemodialyzer (D) to the patient;
first fluid feeding means (P1) disposed in the dialysate supply line (L1);
second fluid feeding means (P2) disposed in the dialysate discharge line (L2);
third fluid feeding means (P3) that can rotate reversibly and is disposed in a bypass line that communicates an upstream side and a downstream side of any one or both of the first fluid feeding means (P1) and the second fluid feeding means (P2) with each other;
a blood pump (P4) disposed in the artery side blood line (L3);
a vein side chamber (C_{V}) including a mesh, disposed in the vein side blood line (L4);
an overflow line (L5) connected to the vein side chamber (C_{V});
and
control means (G2) that conducts a process in which the blood pump (P4) forwardly rotates at a speed lower than a reverse filtration speed made by the third fluid feeding means (P3), and the dialysate that has been pushed into a hollow fiber within the hemodialyzer (D) through a reverse filtering operation made by the third fluid feeding means (P3) circulates in a loop formed by connecting the artery side blood line (L3) and the vein side blood line (L4), which extends from a connection portion between the hemodialyzer (D) and the vein side blood line (L4) to a connection portion between the hemodialyzer (D) and the artery side blood line (L3) through the vein side chamber (C_{V}), to thereby prime the flow passages and the hemodialyzer (D).
